(19) Europäisches Patentamt
European Patent Office
Office européen des brevets

(11) EP 0 647 648 B1

(12) EUROPÄISCHE PATENTSCHRIFT

(45) Veröffentlichungstag und Bekanntmachung des Hinweises auf die Patenterteilung:
**10.03.1999 Patentblatt 1999/10**

(51) Int Cl.[6]: **C07F 9/50**, C07F 9/6568, C07F 15/00 // C07M7/00

(21) Anmeldenummer: **94114992.4**

(22) Anmeldetag: **23.09.1994**

(54) **Optisch aktive phosphorverbindungen**

Optically active phosphorus compounds

Composés de phosphore optiquement actifs

(84) Benannte Vertragsstaaten:
**AT BE CH DE DK ES FR GB IT LI NL**

(30) Priorität: **08.10.1993 CH 3038/93**

(43) Veröffentlichungstag der Anmeldung:
**12.04.1995 Patentblatt 1995/15**

(73) Patentinhaber: **F. HOFFMANN-LA ROCHE AG**
**4002 Basel (CH)**

(72) Erfinder:
- **Broger, Emil Albin**
  **CH-4312 Magden (CH)**
- **Cereghetti, Marco**
  **CH-4058 Basel (CH)**
- **Rageot, Alain**
  **F-68300 Saint-Louis (FR)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:
**WO-A-93/15089**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 647 648 B1

**Beschreibung**

Die vorliegende Erfindung betrifft neue optisch aktive Phosphorverbindungen, welche neben der vorgegebenen axialen Chiralität des Biphenylskelettes zusätzliche Chiralitätszentren an einem oder beiden Phosphoratomen aufweisen, der allgemeinen Formel

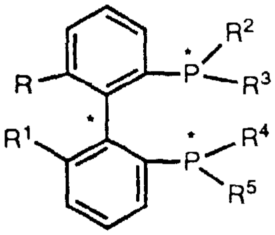

I

worin

R und $R^1$ unabhängig voneinander Hydroxy, eine geschützte Hydroxygruppe, niederes Alkyl oder niederes Alkoxy bedeuten und

$R^2$ bis $R^5$ unabhängig voneinander Alkyl mit 3 bis 7 Kohlenstoffatomen, Cycloalkyl oder Aryl, oder $R^2$ und $R^3$ oder $R^4$ und $R^5$ zusammen mit dem Phosphor eine Gruppe der Formel

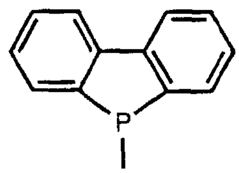

bedeuten; mit der Massgabe, dass nicht gleichzeitig $R^2$ gleich $R^3$ und $R^4$ gleich $R^5$ sind.

Die Erfindung betrifft ferner die Herstellung der Phosphorverbindungen der Formel I, deren Verwendung für enantioselektive Reaktionen, wie z.B. asymmetrische Hydrierungen, enantioselektive Wasserstoffverschiebungen in prochiralen, allylischen System, und dergleichen, sowie Komplexe der Verbindungen der Formel I mit Metallen der Gruppe VIII.

Als Schutzgruppen für die Hydroxygruppe kommen, im Rahmen der vorliegenden Anmeldung insbesondere die üblichen etherbildenden Gruppen, wie z.B. Benzyl, Allyl, Benzyloxymethyl, niederes Alkoxymethyl oder auch 2-Methoxyethoxymethyl in Frage.

Der Ausdruck "niederes Alkyl" bedeutet, im Rahmen der vorliegenden Anmeldung geradkettige oder verzweigte Alkylgruppen mit 1 bis 4 Kohlenstoffatomen, wie z.B. Methyl, Ethyl, Propyl, Isopropyl, n-Butyl, Isobutyl und tert.Butyl. Der Ausdruck "niederes Alkoxy" bedeutet Gruppen in denen der Alkylrest wie oben definiert ist.

Der Ausdruck "Alkyl mit 3 bis 7 Kohlenstoffatomen" bedeutet im vorliegenden Fall geradkettige oder verzweigte Gruppen, wie z.B. Propyl, Isopropyl, n-Butyl, Isobutyl, tert.Butyl, n-Pentyl, Isopentyl und dergleichen. Insbesondere jedoch Isopropyl, Isobutyl oder tert.Butyl. "Cycloalkyl" steht im Rahmen der vorliegenden Erfindung für 3- bis 7-gliedrige Ringe, wie z.B. Cyclopropyl, Cyclobutyl, Cyclopentyl, Cyclohexyl und Cycloheptyl, insbesondere für Cyclopentyl oder Cyclohexyl. Der Ausdruck "Aryl" bedeutet hier insbesondere den Phenylrest, welcher sowohl unsubstituiert, als auch in ortho-, meta- oder para-Stellung oder auch mehrfach substituiert sein kann. Als Substituenten kommen z.B. in Frage Phenyl, niedere Alkyl- oder Alkoxygruppen, vorzugsweise Methyl- oder Methoxygruppen, oder auch Diniederes Alkylamino, vorzugsweise Dimethylamino oder Diethylamino, sowie Chlor, oder auch Trialkylsilyl, wie z.B. Trimethylsilyl. Der Ausdruck kann zudem auch Naphthyl bedeuten. Bevorzugte Arylreste sind insbesondere Phenyl, p-Tolyl, m-Tolyl, m,m'-Dimethylphenyl, m,m'-Diisopropylphenyl, p-Dimethylaminophenyl, p-Methoxyphenyl, m-Methoxyphenyl, p-Methoxy-m,m'-dimethylphenyl, und p-Chlorphenyl.

Die Patentanmeldung WO-A-93/15089 offenbart racemische und optisch aktive Phosphorverbindungen der Formel (1), worin R niederes Alkyl, niederes Alkoxy, Hydroxy oder eine geschützte Hydroxygruppe bedeutet und $R^1$ and $R^2$ voneinander verschieden sind und niederes Alkyl, Cycloalkyl, Aryl, einen fünfgliedrigen Heteroaromaten oder eine Gruppe der Formel ($\alpha$) darstellen. Die Verbindungen der Formel (1) dienen, in Form von Komplexen mit einem

Metall der Gruppe VIII, als Katalysatoren für asymmetrische Hydrierungen und für enantioselektive Wasserstoffverschiebungen in prochiralen allylischen Systemen.

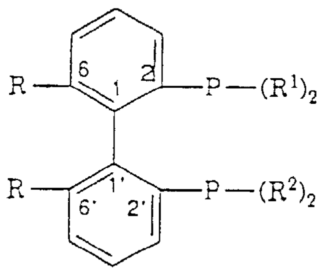

(I)

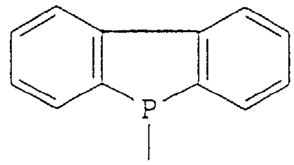

(a)

Während die stereogenen Zentren bei den meisten Liganden entweder nur am Kohlenstoffgerüst, oder nur an den Phosphoratomen lokalisiert sind, wie beispielsweise beim DIPAMP (Ethylen-bis[(S)-(2-methoxyphenyl)phenylphosphin], sind für Liganden die Stereozentren an beiden Stellen enthalten nur wenige Beispiele bekannt. Auch diese werden vor allem zur Komplexbildung mit Übergangsmetallen der Gruppe VIII, insbesondere mit Ruthenium, Rhodium oder Iridium verwendet. Solche DiphosphinMetall-Komplexe werden beispielsweise als Katalysatoren bei asymmetrischen Hydrierungen und auch für enantioselektive Wasserstoffverschiebungen in prochiralen, allylischen Systemen verwendet.

Durch die vorliegende Erfindung werden nun optisch aktive Phosphinliganden mit einer vorgegebenen axialen Chiralität zur Verfügung gestellt, welche sich zusätzlich durch ein oder zwei asymmetrische Phosphoratome auszeichnen. Die Kombination dieser Symmetrieelemente, Chiralitäts- Achse und Chiralitäts- Zentren, in einem Liganden ist neu. Es sind dies die Verbindungen der oben definierten Formel I.

Die Phosphorverbindungen der Formel I können sowohl in racemischer als auch in optisch aktiver Form vorliegen. Bevorzugte Verbindungen der Formel I sind Verbindungen, worin R und $R^1$ die gleiche Bedeutung haben und niederes Alkyl, insbesondere Methyl, oder niederes Alkoxy, insbesondere Methoxy bezeichnen. Solche Verbindungen haben beispielsweise die Formeln

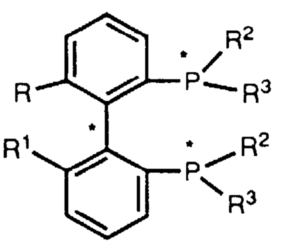

**I-A,**

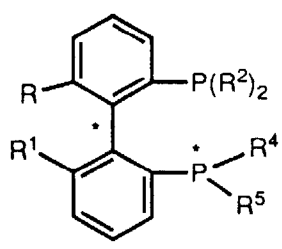

**I-B,**

worin R, $R^1$, $R^2$, $R^3$, $R^4$ und $R^5$ die obengenannten Bedeutungen haben.

Besonders bevorzugte Verbindungen der Formeln I-A und I-B sind diejenigen, worin R und $R^1$ niederes Alkyl oder niederes Alkoxy bedeutet; $R^2$ und $R^4$ unabhängig voneinander Cycloalkyl oder Aryl bedeuten; $R^3$ und $R^5$ unabhängig voneinander Isopropyl, Isobutyl oder tert.Butyl darstellen.

Ganz besonders bevorzugte Verbindungen sind solche der Formeln I-A und I-B in deren optisch aktiven Form wie beispielsweise

(*R,S*)-, (*R,R*)- und (*S,S*)-P-tert.Butyl-P-phenyl-P',P'-diphenyl-(6,6'-dimethylbiphenyl-2,2'-diyl)diphosphin
(*R,S*)-, (*R,R*)- und (*S,S*)-P-Cyclohexyl-P-phenyl-P',P'-diphenyl-(6,6'dimethylbiphenyl-2,2'-diyl)diphosphin
(*R,S*)-, (*R,R*)- und (*S,S*)-P-tert.Butyl-P-phenyl-P',P'-di-p-tolyl-(6,6'-dimethylbiphenyl-2,2'-diyl)diphosphin
(*R,S,S*)-, (*S,S,S*)- und (*R,R,S*)-P,P'-Di-tert.butyl-P,P'-diphenyl-(6,6'-dimethylbiphenyl-2,2-diyl)diphosphin
(R,S,S)-, (S,S,S)- und (R,R,S)-P,P'-Di-cyclohexyl-P,P'-diphenyl-(6,6'-dimethylbiphenyl-2,2-diyl)diphosphin
(*R,S,S*)-, (*S,S,S*)- und (*R,R,S*)-P,P'-Di-β-naphthyl-P,P'-diphenyl-(6,6'-dimethylbiphenyl-2,2-diyl)diphosphin;

Das erfindungsgemässe Verfahren zur Herstellung der Verbindungen der Formel I besteht darin, dass eine chirales 6,6'-disubstituiertes 2,2'-Diiodobiphenyl der Formel

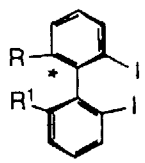

II

worin R und $R^1$ die obengenannten Bedeutungen haben,

zunächst mit tert.Butyllithium und anschliessend mit je einem entsprechend substituierten, racemischen Chlorphosphin-Derivat der Formel

$$ClPR^2R^3 \quad \underline{A}$$

und

$$ClPR^4R^5 \quad \underline{B}$$

worin $R^2$ bis $R^5$ die obengenannten Bedeutungen haben,

oder aber mit zwei Chlorphosphin-Derivaten der Formel A umgesetzt wird, bzw. ein 2-Monoiodobiphenyl Derivat der Formel

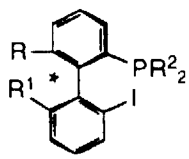

III

zunächst mit tert.Butyllithium und anschliessend mit einem entsprechend substituierten racemischen Chlorphosphin der Formel B umsetzt wird.

Schema 1

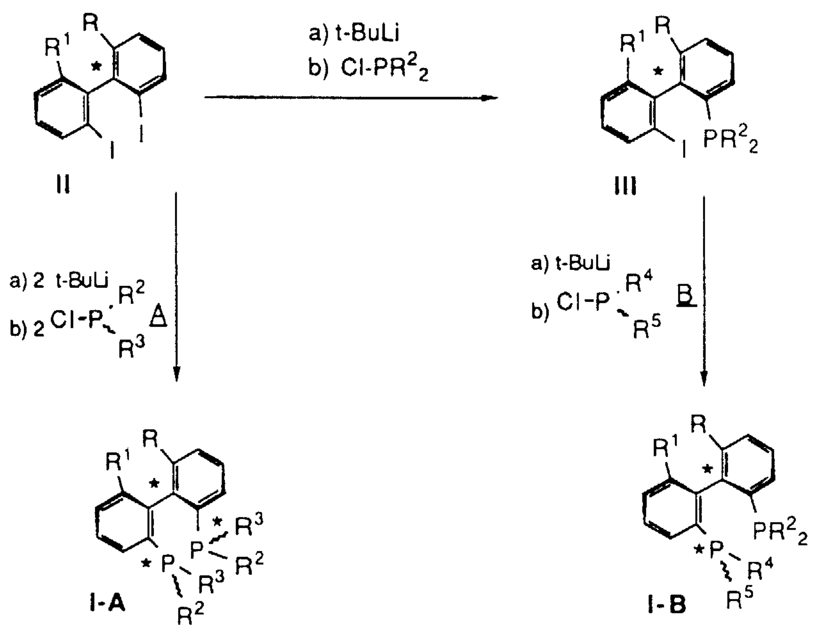


**3 Diastereomere**
chromatographisch trennbar
axial chirales Biphenyl und
Chiralitätszentrum an beiden P*

**2 Diastereomere**
chromatographisch trennbar
axial chirales Biphenyl und
Chiralitätszentrum an einem P*

Der Vorteil dieser Reaktionsführung besteht darin, dass die gewählte Konfiguration des Biphenyls bei der Umsetzung unverändert beibehalten wird. Dadurch sind maximal nur drei bzw. zwei Diastereomere möglich (I-A bzw. I-B; Schema 1). Dabei wird jeweils, bedingt durch sterische Interaktionen zwischen dem asymmetrischen Biphenyl-Templat und den beiden organischen Resten am Phosphor, direkt oder nach erfolgter thermischer Aequilibrierung, eines der Diastereomeren bevorzugt gebildet.

Ausgehend von Verbindungen der Formel II können somit durch das erfindungsgemässe Verfahren, Verbindungen der Formel I und I-B in zwei Stufen und Verbindungen der Formel I-A in einer einzigen Stufe hergestellt werden.

Für die Herstellung der Verbindungen der Formel I-A wird ein Diiododiphenyl-Derivat der Formel II zunächst mit tert.Butyllithium in einem geeigneten Lösungsmittel in die entsprechende metallierte Verbindung übergeführt und anschliessend mit zwei entsprechend substituierten racemischen Chlorphosphin-Derivaten A umgesetzt. Das anfallende Produktgemisch aus drei Diastereoisomeren wird durch Chromatographie in die chiralen Einzelkomponenten aufgetrennt.

Die Verbindungen der Formel I-B werden dadurch hergestellt, dass das als Ausgangsverbindung verwendete Diiodobiphenyl Derivat der Formel II zunächst wie oben beschrieben mit einem Aequivalent tert.Butyllithium in die entsprechende metallierte Verbindungen übergeführt werden und anschliessend mit einem Aequivalent des disubstituierten Chlorphosphin ($ClP(R^2)_2$ in eine Verbindung der Formel III umgesetzt wird, welche dann, nochmals metalliert, mit racemischen Chlorphosphin der Formel B umgesetzt wird. Die zwei Diastereomere der Formel I-B können chromatographisch getrennt werden.

Zur Herstellung der Verbindungen der Formel I wird das als Ausgangsmaterial verwendete Diiodid der Formel II zunächst monometalliert und mit einem Aequivalent des racemischen Chlorphosphins der Formel A und anschliessend, erneut metalliert, mit einem Aequivalent des racemischen Chlorphosphins der Formel B umgesetzt.

Die Umsetzung der metallierten Derivate der Verbindungen II bzw. III mit Chlorphosphin erfolgt zweckmässig unter Inertgas, wie z.B. Argon oder Stickstoff, in einem inerten Lösungsmittel wie beispielsweise THF, Ether, oder einem Gemisch des einen oder beider mit Toluol bei Temperaturen von ca. -78 bis maximal -50° vorzugsweise bei ca. -70° - ca. -55°.

Die Verbindungen der Formel II und III sind bekannte Verbindungen und können auf an sich bekannte Weise,

wie z.B. in WO 93/15090 beschrieben, hergestellt werden.

Die erfindungsgemässen Phosphorverbindungen der Formel I bilden Komplexe mit Uebergangsmetallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium- und Rhodium-Komplexe bevorzugt, während für Isomerisierungen Rhodium-Komplexe bevorzugt sind. Diese Katalysatoren, dh. die Komplexe aus einem Metall der Gruppe VIII und den Phosphorverbindungen der Formel I sind neu und ebenfalls Gegenstand der vorliegenden Erfindung.

Die in Frage stehenden Komplexe können in an sich bekannter Weise hergestellt werden, beispielsweise wie in EP 538 336 beschrieben.

Die nachfolgenden Beispiele dienen zur Illustrierung der Erfindung und sollen in keiner Weise eine Beschränkung darstellen. In den Beispielen haben die gewählten Ausdrücke die folgende Bedeutung:

| | |
|---|---|
| GC | Gaschromatographie |
| GC-Fl% | GC-Flächenprozent |
| HPLC | Hochdruck-Flüssigchromatographie |
| e.e. | Enantiomeren Überschuss (Enantiomeric Excess) |
| RT | Raumtemperatur |

Alle Temperaturen sind in °Celsius angegeben

Beispiel 1

Herstellung von *(R,S)*-, *(R,R)*- und *(S,S)*-P-tert.Butyl-P-phenyl-P',P'-diphenyl-(6,6'-dimethylbiphenyl-2,2'-diyl)diphosphin

In einem Gemisch aus 200 ml absolutem Toluol und 50 ml Ether wurden unter Ar-Begasung 14.4 g (0.029 Mol) *(S)*-Diphenyl-(2'-iod-6,6'-dimethylbiphenyl-2-yl)phosphin gelöst. Nach Abkühlen auf -70° wurden 30 ml Butyllithium-Lösung (1.6 M in Hexan; 0.048 Mol) zugegeben und das Reaktionsgemisch 45 Min.bei -69° gerührt. Anschliessend wurden bei -65° innert 15 Min.16.0 g (0.080 Mol) *(rac)*-tert.Butylphenylchlorphosphin, gelöst in 50 ml Toluol, zugetropft, und die grau-beige Suspension 1 Std.bei -65°, dann über Nacht bei RT gerührt.

Zur Aufarbeitung wurde das Gemisch mit 85 ml Wasser und 30 ml 3N NaOH versetzt, 15 Min. gerührt, mit 300 ml Toluol extrahiert, die organische Phase zweimal mit 150 ml Wasser gewaschen, getrocknet ($Na_2SO_4$), filtriert und eingedampft. Nach einer Chromatographie an 400 g Kieselgel (Hexan-Toluol 1:4 - 1:1; bis Toluol ) und Umkristallisation der beiden Rohfraktionen [*(R,S)*-P-tert.Butyl-P-phenyl-P',P'-diphenyl-(6,6'-dimethylbiphenyl-2,2'-diyl)diphosphin: 7.3 g (47% d.Th), und *(S,S)*-P-tert.Butyl-P-phenyl-P',P'-diphenyl-(6,6'-dimethylbiphenyl-2,2'-diyl)diphosphin: 3.9 g (25% d.Th)] aus AcOEt/MeOH, resultierten

3.5 g (23% d.Th.) *(R,S)*-P-tert.Butyl-P-phenyl-P',P'-diphenyl-(6,6'-dimethylbiphenyl-2,2'-diyl)diphosphin, als weisse Kristalle, Smp.: 140.9°; (100% e.e., gemäss HPLC-Analyse an einer Chiracel OD-Phase; GC-Gehalt: 99.5%); $[a]_D^{20}$ + 154.3° (c=1; $CHCl_3$) und
2.3 g (15% d.Th.) *(S,S)*-P-tert.Butyl-P-phenyl-P',P'-diphenyl-(6,6'-dimethylbiphenyl-2,2'-diyl)diphosphin, als weisse Kristalle, Smp.: 142.6°; (100% e.e., gemäss HPLC-Analyse an einer Chiracel OD-Phase; GC-Gehalt: 97%); $[a]_D^{20}$ - 121.1° (c=1; $CHCl_3$).

Auf analoge Weise, unter Verwendung von (R)-Diphenyl-(2'-iod-6,6'-dimethylbiphenyl-2-yl)phosphin wurden folgende Verbindungen hergestellt:

(*R,R*)-P-tert.Butyl-P-phenyl-P',P'-diphenyl-(6,6'-dimethylbiphenyl-2,2'-diyl)diphosphin; Smp.: 147-149°;
(*S,R*)-)-P-tert.Butyl-P-phenyl-P' ,P'-diphenyl-(6,6'-dimethylbiphenyl-2'2'-diyl)diphosphin.

Beispiel 2

Auf analoge Weise wie in Beispiel 1, unter Verwendung von *(R)*-Diphenyl-(2'-iod-6,6'-dimethylbiphenyl-2-yl) phosphin, aber von *(rac)*-Cyclohexylphenylchlorphosphin an Stelle von *(rac)*-tert.Butylphenylchlorphosphin, wurde folgende Verbindung hergestellt:

(*R,R*)-P-Cyclohexyl-P-phenyl-P',P'-diphenyl-(6,6'-dimethylbiphenyl-2,2'-diyl)diphosphin; Smp.: 187.3-188.1°

Beispiel 3

In zu Beispiel 1 analoger Weise, unter Verwendung von *(S)*-Di-p-tolyl-(2'-iod-6,6'-dime thylbiphenyl-2-yl)phosphin wurden folgende Verbindungen hergestellt:

*(R,S)*-P-tert.Butyl-P-phenyl-P',P'-di-p-tolyl-(6,6'-dimethylbiphenyl-2,2'-diyl)diphosphin; Smp.: 161.5-162°; $[a]_D^{20}$ +168.6° (c=0.8; $CHCl_3$), und
*(S,S)*-P-tert.Butyl-P-phenyl-P',P'-di-p-tolyl-(6,6'-dimethylbiphenyl-2,2'-diyl)diphosphin.

Beispiel 4

Das in den Beispielen 1 und 2 als Ausgangsmaterial verwendete (S)-Diphenyl-(2'-iod-6,6'-dimethyl-1,1'-biphenyl-2-yl)phosphin wurde wie folgt hergestellt:

Zu einer auf -76° gekühlten Lösung von 12.6 g (0.029 Mol) (S)-2,2'-Diiod-6,6'-dimethyl-1,1'-biphenyl, gelöst in 200 ml absolutem Toluol und 50 ml Ether wurde unter Ar-Begasung 18 ml einer tert.Butyllithium-Lösung (15%ige Lösung in Pentan; 0.028 mol) zugegeben und das Gemisch 1 Std.bei -70° gerührt. Aus einem Tropftrichter wurden anschliessend innert 15 Min.eine Lösung von 13 g (0.062 Mol) Chlordiphenylphosphin in 50 ml absolutem Toluol zugetropft , das Gemisch 1 Std.bei ca. -70°, und nach dem Entfernen des Kühlbades 1 Std. bei RT gerührt.

Zur Aufarbeitung wurde das Reaktionsgemisch, eine grau-beige Suspension, mit 70 ml Wasser versetzt, mit 30 ml 3N NaOH alkalisch gestellt und mit 500 ml Essigester extrahiert. Die organische Phase wurde mit 150 ml Wasser neutral gewaschen, getrocknet ($Na_2SO_4$), eingedampft und der resultierende Rückstand (26 g; gelbes Oel) an 300 g Kieselgel chromatographiert.

In zwei Hauptfraktionen eluierte man zunächst mit 2.1 l Hexan-Toluol 6:4-Gemisch 3.7 g unverändertes Ausgangsmaterial (GC-Gehalt: 75%), und anschliessend mit 3.5 l Hexan-Toluol (1:1)-Gemisch 9.2 g (75%, d.Th.) enantiomerenreines *(S)*-Diphenyl-(2'-iod-6,6'-dimethyl-1,1'-biphenyl-2-yl)phosphin (HPLC: 99.8% ee) , als weisse Kristalle, die direkt in die nächstfolgende Stufe eingesetzt wurden. Kristallisation aus AcOEt/MeOH ergab reines *(S)-Diphenyl-(2'-iod-6,6'-dimethyl-1,1'-biphenyl-2-yl)phosphin*, Smp.: 167.5-168.4°; $[\alpha]_D^{20}$ + 43.5° (c=1; $CHCl_3$).

Auf analoge Weise wurden folgende Ausgangsmaterialien hergestellt:

*(rac)*-Diphenyl-(2'-iod-6,6'-dimethyl-1,1'-biphenyl-2-yl)phosphin;
*(R)*-Diphenyl-(2'-iod-6,6'-dimethyl-1,1'-biphenyl-2-yl)phosphin;
*(S)*-Di-p-tolyl-(2'-iod-6,6'-dimethyl-1,1'-biphenyl-2-yl)phosphin;
*(S)*-Di-cyclohexyl-(2'-iod-6,6'-dimethyl-1,1'-biphenyl-2-yl)phosphin;
*(S)*-Di-benzophosphol(2'-iod-6,6'-dimethyl-1,1'-biphenyl-2-yl)phosphin;
*(S)*-Diphenyl-(2'-iod-6,6'-dimethoxy-1,1'-biphenyl-2-yl)phosphin;

Smp.: 125.7°

*(rac)*-Diphenyl-(2'-iod-6,6'-dimethoxy-1,1'-biphenyl-2-yl)phosphin;

Smp.: 194.0 - 194.4°.

Beispiel 5

In zu Beispiel 1 analoger Weise, unter Verwendung von *(S)*-2,2'-Diiod-6,6'-dimethyl-1,1'-biphenyl und je der doppelten Menge an BuLi und dem entsprechenden Chlorphosphin, wurden die Liganden der allgemeinen Formel I-A mit zwei chiralen P*-Atomen hergestellt:

*(R,R,S)*-P,P'-Di-tert.Butyl-P,P'-diphenyl-(6,6'-dimethylbiphenyl-2,2-diyl)diphosphin; Smp.: 138.8°; (99.1% d.e.; GC: 97%); $[a]_D^{20}$ + 183.5° (c=0.4; $CHCl_3$).
*(R,S,S)*-P,P'-Di-tert.Butyl-P,P'-diphenyl-(6,6'-dimethylbiphenyl-2,2-di-yl)diphosphin; Smp.: 120.1° (99% d.e.; GC: 98.1%); $[a]_D^{20}$ + 34.6° (c=0.8; $CHCl_3$).
*(S,S,S)*-P,P'-Di-tert.Butyl-P,P'-diphenyl-(6,6'-dimethylbiphenyl-2,2-diyl)diphosphin; Smp.: 143.3°; (99% d.e.; GC: 99.5%); $[\alpha]_D^{20}$ - 246.0° (c=1; $CHCl_3$).
*(R,R,S)*-P,P'-Di-cyclohexyl-P,P'-diphenyl-(6,6'-dimethylbiphenyl-2,2-diyl)diphosphin; Smp.: 190°; (98% d.e.; GC: 98%); $[\alpha]_D^{20}$ + 160.7°, (c=0.8; $CHCl_3$).
*(S,S,S)*-P,P'-Di-cyclohexyl-P,P'-diphenyl-(6,6'-dimethylbiphenyl-2,2-diyl)diphosphin; Smp.: 159-190° (98% d.e.; GC: 95%); $[a]_D^{20}$ -56° (c=0.5; $CHCl_3$).

*(R,R,S)*-P,P'-Di-β-naphthyl-P,P'-diphenyl-(6,6'-dimethylbiphenyl-2,2-diyl)diphosphin; Smp.: 169-170°; (85% d.e.; GC: 98%).

Beispiel 6

In einer Glove-Box ($O_2$-Gehalt <1 pm) wurden in einem 100 ml Messkolben 6.4 mg (0.0073 mMol) Tetra-μ-trifluoracetato-bis(1,5-cyclooctadien)diruthenium(II) und 8.1 mg (0.0146 mMol) (R,S)-P-Cyclohexyl-P-phenyl-P',P'-diphenyl-(6,6'-dimethylbiphenyl-2,2'-diyl)diphosphin in 100 ml Methanol unter Rühren bei RT gelöst. Innert 16 Stunden bildete sich sich eine orangerote, klare Katalysatorlösung.

Beispiel 7

In einer Glove-Box ($O_2$-Gehalt <1 pm) wurde ein 500 ml-Autoklav mit 27.0 g (175.0 mMol) Geraniol, 36 ml Methanol und 100 ml nach Beispiel 6 hergestellter Katalysatorlösung beladen. Die Hydrierung wurde bei 20°C, einem konstanten Druck von 60 bar $H_2$ und unter intensivem Rühren durchgeführt. Nach 22 Stunden betrug der Umsatz 100%. Die hellgelbe Hydrierlösung wurde aus dem Autoklaven gespült und am Rotationsverdampfer bei 60°/17 mbar eingedampft. Der Rückstand wurde bei 65°/0.01 mbar destilliert. Man erhielt 26.7 g (99,0%) (R)-Citronellol, als farbloses Oel, mit einer enantioneren Reinheit von 74.3% e.e.. Die Bestimmung des e.e.-Wertes erfolgte durch GC Analyse der mit Trolox™ Methylether hergestellten diastereomeren Ester.

Beispiel 8

In einer Glove-Box ($O_2$-Gehalt <1 pm) wurden in einem 50 ml Messkolben 10.3 mg (0.0315 mMol) Bis(η-2-acetato)(η-4-1,5-cyclooctadien)ruthenium(II) und 17.6 mg (0.0315 mMol) (R,S)-P-Cyclohexyl-P-phenyl-P',P'-diphenyl-(6,6'-dimethylbiphenyl-2,2'-diyl)diphosphin in einem Gemisch von 6 ml Diethylether und 2 ml Tetrahydrofuran gelöst, 16 Stunden bei 40° gerührt und anschliessend mit ca. 42 ml Methanol auf die Marke aufgefüllt. Es bildete sich eine orangerote, klare Katalysatorlösung.

Beispiel 9

In einer Glove-Box ($O_2$-Gehalt <1 pm) wurde ein 500 ml-Autoklav mit 15.0 g (50.44 mMol) (Z)-2-Acetyl-l-(p-methoxybenzyliden)-1,2,3,4,5,6,7,8-octahydroisochinolin, 160 ml Methanol und 10 ml der gemäss Beispiel 8 hergestellten Katalysatorlösung beladen. Die Hydrierung erfolgte bei 100° und einem konstanten Druck von 35 bar $H_2$ unter intensivem Rühren. Nach 22 Stunden betrug der Umsatz 74.0%. Ein aliquoter Teil der Hydrierlösung (enthaltend ca 2 g rohes Produkt wurde bei 40°/17 mbar eingedampft. Der Rückstand (1.95 g), bestand aus einem Gemisch von 74 GC-Fl% (S)-2-Acetyl-1-(p-methoxybenzyl)-1,2,3,4,5,6,7,8-octahydroisochinolin: e.e. 91.2 % und aus 26% nicht umgesetzten Edukt.

Zur Bestimmung des e.e.-Wertes wurde eine homogene Probe des Rückstandes in einem Gemisch von Ethylenglykol und 40 proz. wässeriger KOH-Lösung während 18 Stunden bei 170°C hydrolysiert. Das resultierende Amin wurde in Pyridin/4-Dimethylamino-pyridin mit (-)-Camphansäurechlorid ins Gemisch der diastereomeren Amide übergeführt, welche gaschromatographisch auf einer PVMS-54 Kapillarsäule analysiert wurden.

Beispiel 10

In einer Glove-Box ($O_2$-Gehalt <1 pm) wurde ein 500 ml ml-Autoklav mit 23.1 g (90.0 mMol) 3-Oxotetradecansäure-methylester, 60 ml Methanol und einer Lösung von 5.4 mg (0.009 mMol) $Ru_2(COD)_2Cl(\mu Cl)_3(CH_3CN)$ (Lit.: E. Singleton et al., S.-Afr. Tydskr. Chem., 40, 183 (1987) und 10.0 mg (0.018 mMol) (R,S)-P-Cyclohexyl-P-phenyl-P',P'-diphenyl-(6,6'-dimethylbiphenyl-2,2'-diyl)diphosphin in 5 ml Dichlormethan beladen. Die Hydrierung erfolgte bei 80° und 35 bar. Nach 22 Stunden betrug der Umsatz 100%.

Die Hydrierlösung wurden bei 50°/17 mbar eingeengt und der kristalline Rückstand in 250 ml Diethylether gelöst. Zur Abtrennung des Katalysators wurde die Etherlösung über 50 g Kieselgel filtriert. Nach dem Einengen und Trocknen der filtrierten Etherphase resultierten 22.9 g (S)-3-Hydroxytetradecansäure-methylester als weisses Kristalle: e.e. 88.5%. Zur e.e.-Bestimmung wurde eine homogene Probe mit (S)-6-Methoxy-2,5,7,8-tetramethylchroman-2-carbonsäure verestert und die Diastereomeren gaschromatographisch auf einer OV-240-OH Kapillarsäule analysiert.

## Patentansprüche

**1.** Verbindungen der allgemeinen Formel

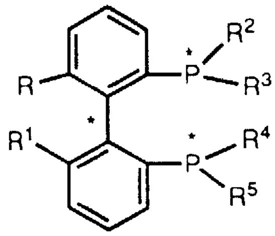

I

worin

R und $R^1$ unabhängig voneinander Hydroxy, eine geschützte Hydroxygruppe, niederes Alkyl oder niederes Alkoxy bedeuten und

$R^2$ bis $R^5$ unabhängig voneinander Alkyl mit 3 bis 7 Kohlenstoffatomen, Cycloalkyl oder Aryl, oder $R^2$ und $R^3$ oder $R^4$ und $R^5$ zusammen mit dem Phosphor eine Gruppe der Formel

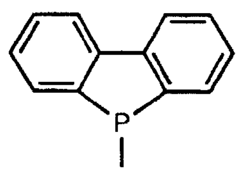

bedeuten; mit der Massgabe, dass nicht gleichzeitig $R^2$ gleich $R^3$ und $R^4$ gleich $R^5$ sind.

**2.** Verbindungen gemäss Anspruch 1, der allgemeinen Formeln

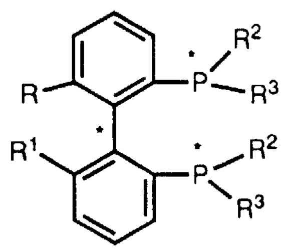

I-A,

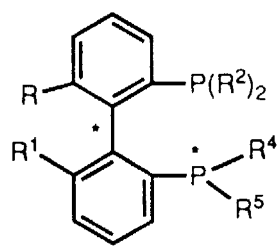

I-B,

worin R, $R^1$, $R^2$, $R^3$, $R^4$ und $R^5$ die in Anspruch 1 genannten Bedeutungen haben.

**3.** Verbindungen gemäss einem der Ansprüche 1 oder 2, dadurch gekennzeichnet, dass R und $R^1$ die gleiche Bedeutung haben und niederes Alkyl, insbesondere Methyl, oder niederes Alkoxy, insbesondere Methoxy bezeichnen.

**4.** Verbindungen gemäss einem der Ansprüche 1 bis 3, dadurch gekennzeichnet, dass $R^2$ und $R^4$ unabhängig voneinander Cycloalkyl oder Aryl bedeuten; und $R^3$ und $R^5$ unabhängig voneinander Isopropyl, Isobutyl oder tert.Butyl darstellen.

**5.** *(R,S)-*, *(R,R)-* und (S,S)-P-tert.Butyl-P-phenyl-P',P'-diphenyl-(6,6'-dimethylbiphenyl-2,2'-diyl)diphosphin.

**6.** *(R,S)-, (R,R)-* und (S,S)-P-Cyclohexyl-P-phenyl-P',P'-diphenyl-(6,6'-dimethylbiphenyl-2,2'-diyl)diphosphin.

**7.** *(R,S)-*, *(R,R)-* und *(S,S)-*P-tert.Butyl-P-phenyl-P',P'-di-p-tolyl-(6,6'-dimethylbiphenyl-2,2'-diyl)diphosphin.

**8.** *(R,S,S)-*, *(S,S,S)-* und *(R,R,S)-*P,P'-Di-tert.Butyl-P,P'-diphenyl-(6,6'-dimethylbiphenyl-2,2-diyl)diphosphin.

**9.** *(R,S,S)-*, *(S,S,S)-* und *(R,R,S)-*P,P'-Di-cyclohexyl-P,P'-diphenyl-(6,6'-dimethylbiphenyl-2,2-diyl)diphosphin.

**10.** *(R,S,S)-*, *(S,S,S)-* und (R,R,S)-P,P'-Di-β-naphthyl-P,P'-diphenyl-(6,6'-dimethylbiphenyl-2,2-diyl)diphosphin.

**11.** Verfahren zur Herstellung von Verbindungen der allgemeinen Formel

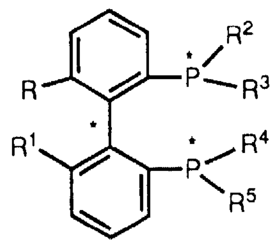

I

worin

R und $R^1$ unabhängig voneinander Hydroxy, eine geschützte Hydroxygruppe, niederes Alkyl oder niederes Alkoxy bedeuten und

$R^2$ bis $R^5$ unabhängig voneinander Alkyl mit 3 bis 7 Kohlenstoffatomen, Cycloalkyl oder Aryl, oder $R^2$ und $R^3$ oder $R^4$ und $R^5$ zusammen mit dem Phosphor eine Gruppe der Formel

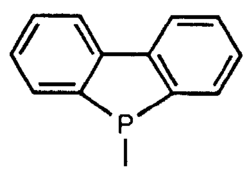

bedeuten; mit der Massgabe, dass nicht gleichzeitig $R^2$ gleich $R^3$ und $R^4$ gleich $R^5$ sind,

dadurch gekennzeichnet, dass

a) eine chirale Verbindung der Formel

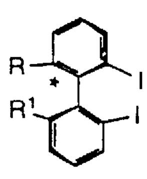

II

worin R und $R^1$ die oben angegebene Bedeutung haben;
zunächst mit tert.Butyllithium und anschliessend mit je einem entsprechend substituierten, racemischen Chlorphosphin-Derivat der Formeln

$ClPR^2R^3$ A

und

$ClPR^4R^5$ B

worin $R^2$ bis $R^5$ wie die obige Bedeutung haben; umgesetzt wird, oder

b) eine Verbindung der Formel II zunächst mit tert.Butyllithium und anschliessend mit zwei Chlorphosphin-Derivaten der Formel A umgesetzt wird, oder

c) ein chirales 2-Monoiodobiphenyl-Derivat der Formel

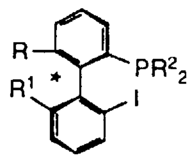

III

worin R, $R^1$ und $R^2$ die obengenannten Bedeutungen haben, zunächst mit tert.Butyllithium und anschliessend mit einem entsprechend substituierten, racemischen Chlorphosphin-Derivat der Formel B umgesetzt wird.

**Claims**

1. Compounds of the general formula

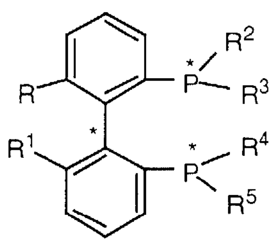

I

wherein

R and $R^1$ each independently signify hydroxy, a protected hydroxy group, lower alkyl or lower alkoxy and

$R^2$ to $R^5$ each independently signify alkyl with 3 to 7 carbon atoms, cycloalkyl or aryl or $R^2$ and $R^3$ or $R^4$ and $R^5$ together with the phosphorus form a group of the formula

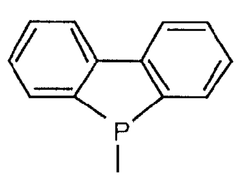

;

with the proviso that simultaneously $R^2$ is not the same as $R^3$ and $R^4$ is not the same as $R^5$.

2. Compounds according to claim 1 of the general formulae

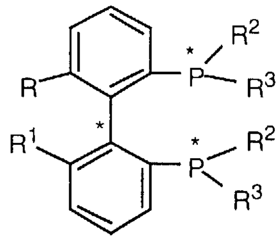 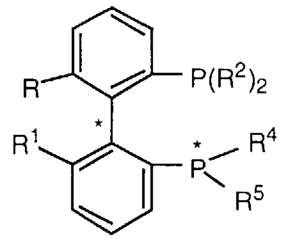

I-A, I-B,

wherein R, $R^1$, $R^2$, $R^3$, $R^4$ and $R^5$ have the significances set forth in claim 1.

**3.** Compounds according to claim 1 or 2, characterized in that R and $R^1$ have the same significance and denote lower alkyl, especially methyl, or lower alkoxy, especially methoxy.

**4.** Compounds according to any one of claims 1 to 3, characterized in that $R^2$ and $R^4$ each independently signify cycloalkyl or aryl; and $R^3$ and $R^5$ each independently represent isopropyl, isobutyl or tert.butyl.

**5.** *(R,S)*-, *(R,R)*- and *(S,S)*-P-tert.Butyl-P-phenyl-P',P'-diphenyl-(6,6'-dimethylbiphenyl-2,2'-diyl)diphosphine.

**6.** *(R,S)-, (R,R)-* and *(S,S)*-P-Cyclohexyl-P-phenyl-P',P'-diphenyl-(6,6'-dimethylbiphenyl-2,2'-diyl)diphosphine.

**7.** *(R,S)*-, *(R,R)*- and *(S,S)*-P-tert.Butyl-P-phenyl-P',P'-di-p-tolyl-(6,6'-dimethylbiphenyl-2,2'-diyl)diphosphine.

**8.** *(R,S,S)*-, *(S,S,S)*- and *(R,R,S)*-P,P'-di-tert.Butyl-P,P'-diphenyl-(6,6'-dimethylbiphenyl-2,2-diyl)diphosphine.

**9.** *(R,S,S)*-, *(S,S,S)*- and *(R,R,S)*-P,P'-di-Cyclohexyl-P,P'-diphenyl-(6,6'-dimethylbiphenyl-2,2-diyl)diphosphine.

**10.** *(R,S,S)*-, *(S,S,S)*- and *(R,R,S)*-P,P'-di-β-Naphthyl-P,P'-diphenyl(6,6'-dimethylbiphenyl-2,2-diyl)diphosphine.

**11.** A process for the manufacture of compounds of the general formula

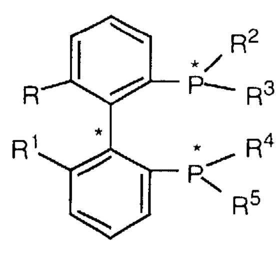

I

wherein

R and $R^1$ each independently signify hydroxy, a protected hydroxy group, lower alkyl or lower alkoxy and

$R^2$ to $R^5$ each independently signify alkyl with 3 to 7 carbon atoms, cycloalkyl or aryl or $R^2$ and $R^3$ or $R^4$ and $R^5$ together with the phosphorus form a group of the formula

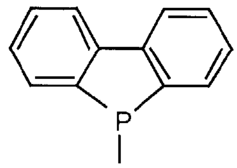 ;

with the proviso that simultaneously $R^2$ is not the same as $R^3$ and $R^4$ is not the same as $R^5$,

characterized by

a) reacting a chiral compound of the formula

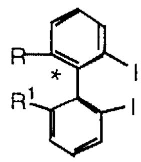 II

wherein R and $R^1$ have the significance given above;
firstly with tert.butyllithium and subsequently with in each case a correspondingly substituted, racemic chlorophosphine derivative of the formulae

$ClPR^2R^3$ A

and

$ClPR^4R^5$ B

wherein $R^2$ to $R^5$ have the above significance,
or

b) reacting a compound of formula II firstly with tert.butyllithium and subsequently with two chlorophosphine derivaties of formula A, or

c) reacting a chiral 2-monoiodobiphenyl derivative of the formula

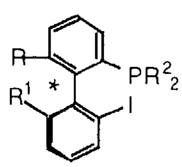 III

wherein R, $R^1$ and $R^2$ have the aforementioned significances,
firstly with tert.butyllithium and subsequently with a correspondingly substituted, racemic chlorophosphine derivative of formula B.

**Revendications**

1. Composés de formule générale

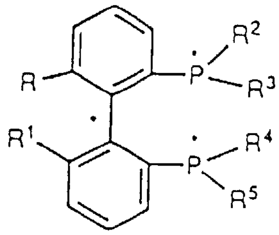

I

dans laquelle

R et $R^1$ représentent indépendamment l'un de l'autre un groupe hydroxy, un groupe hydroxy protégé, un groupe alkyle inférieur ou alcoxy inférieur et

$R^2$ à $R^5$ représentent indépendamment l'un de l'autre un groupe alkyle avec 3 à 7 atomes de carbone, cycloalkyle ou aryle, ou $R^2$ et $R^3$ ou $R^4$ et $R^5$ représentent ensemble avec l'atome de phosphore un groupe de formule

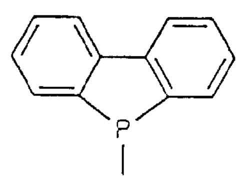

;

à la condition que $R^2$ ne soit pas égal à $R^3$ et $R^4$ égal à $R^5$ en même temps.

2. Composés selon la revendication 1, de formules générales

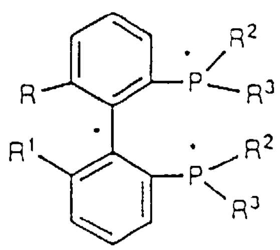

I-A,

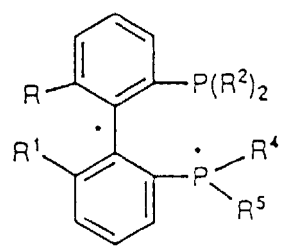

I-B,

dans lesquelles R, $R^1$, $R^2$, $R^3$, $R^4$ et $R^5$ ont les significations mentionnées à la revendication 1.

3. Composés selon l'une des revendications 1 ou 2, caractérisés en ce que R et $R^1$ ont la même signification et désignent un groupe alkyle inférieur, en particulier méthyle, ou un alcoxy inférieur, en particulier un groupe méthoxy.

4. Composés selon l'une des revendications 1 à 3, caractérisées en ce que $R^2$ et $R^4$ représentent indépendamment l'un de l'autre un groupe cycloalkyle ou aryle; et $R^3$ et $R^5$ représentent indépendamment l'un de l'autre un groupe un groupe isopropyle, isobutyle ou tert.-butyle.

5. *(R,S)*-, *(R,R)*-, et *(S,S)*-P-tert.-butyl-P-phényl-P',P'-diphényl-(6,6'-diméthylbiphényl-2,2'-diyl)-diphosphine.

6. *(R,S)-, (R,R)-,* et *(S,S)*-P-cyclohexyl-P-phényl-P',P'-diphényl-(6,6'-diméthylbiphényl-2,2'-diyl)-diphosphine.

**7.** *(R,S)*-, *(R,R)*-, et *(S,S)*-P-tert.-butyl-P-phényl-P', P'-di-p-tolyl-(6,6'-diméthylbiphényl-2,2'-diyl)diphosphine.

**8.** *(R,S,S)*-, *(S,S,S)*-, et *(R,R,S)*-P,P'-di-tert.-butyl-P,P'-diphényl-(6,6'-diméthylbiphényl-2,2'-diyl)-diphosphine.

**9.** *(R,S,S)*-, *(S,S,S)*-, et *(R,R,S)*-P,P'-di-cyclohexyl-P,P'-diphényl-(6,6'-diméthylbiphényl-2,2'-diyl)-diphosphine.

**10.** *(R,S,S)*-, *(S,S,S)* -, et *(R,R,S)* -P, P'-di-β-naphtyl-P,P'-diphényl-(6,6'-diméthylbiphényl-2,2'-diyl)diphosphine.

**11.** Procédé de préparation de composés de formule générale

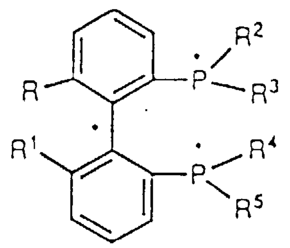

I

dans laquelle

R et $R^1$ représentent indépendamment l'un de l'autre un groupe hydroxy, un groupe hydroxy protégé, un groupe alkyle inférieur ou alcoxy inférieur en

$R^2$ à $R^5$ représentent indépendamment l'un de l'autre un groupe alkyle avec 3 à 7 atomes de carbone, cycloalkyle ou aryle, ou $R^2$ et $R^3$ ou $R^4$ et $R^5$ représentent ensemble avec l'atome de phosphore un groupe de formule

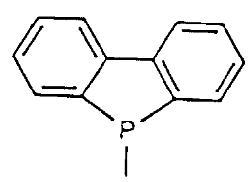

;

à la condition que $R^2$ ne soit pas égal à $R^3$ et $R^4$ égal à $R^5$ en même temps,
caractérisé en ce que l'on met à réagir

a) un composé chiral de formule

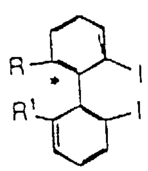

II

dans laquelle R et $R^1$ ont la signification donnée ci-dessus;
d'abord avec le tert.-butyllithium et ensuite avec respectivement un dérivé chlorophosphine racémique, substitué en conséquence de formules

$ClPR^2R^3$ A

et

$ClPR^4R^5$ B

dans lesquelles $R^2$ à $R^5$ ont la signification ci-dessus mentionnée; ou,

b) un composé de formule II d'abord avec le tert.-butyllithium et ensuite avec deux dérivés chlorophosphine de formule A, ou

c) un dérivé 2-monoiodobiphényle chiral de formule

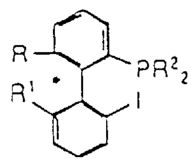

III

dans laquelle R, $R^1$ et $R^2$ ont les significations mentionnées ci-dessus, d'abord avec le tert.-butyllithium et ensuite avec un dérivé chlorophosphine racémique, substitué en conséquence de formule B.